(19) Europäisches Patentamt
European Patent Office
Office européen des brevets

(11) Veröffentlichungsnummer: **0 165 274**
**B1**

(12) # EUROPÄISCHE PATENTSCHRIFT

(45) Veröffentlichungstag der Patentschrift: **01.03.89**

(51) Int. Cl.⁴: **A 61 K 31/195**, A 61 K 31/13

(21) Anmeldenummer: **85900088.7**

(22) Anmeldetag: **27.11.84**

(86) Internationale Anmeldenummer: **PCT/EP 84/00371**

(87) Internationale Veröffentlichungsnummer: **WO 85/02340 (06.06.85 Gazette 85/13)**

(54) **VERWENDUNG VON CYSTEIN-DERIVATEN ODER DEREN SALZEN, ZUR STEIGERUNG DER INSULINSEKRETION DER LANGERHANS'SCHEN INSELN DER BAUCHSPEICHELDRÜSE.**

(30) Priorität: **29.11.83 DE 3343141**

(43) Veröffentlichungstag der Anmeldung: **27.12.85 Patentblatt 85/52**

(45) Bekanntmachung des Hinweises auf die Patenterteilung: **01.03.89 Patentblatt 89/9**

(84) Benannte Vertragsstaaten: **AT BE CH DE FR GB LI LU NL SE**

(56) Entgegenhaltungen:
**DE-A-2 835 000**

**Chemical Abstracts, vol. 71, no. 23, 8. December 1969, Columbus, Ohio, (US), Chiba Takehisa: "Effect of sulfur-containing compounds on experimental diabetes. VI. Screening for hypo-glycemic action of sulfur-containing compounds", p. 200, abstract no. 111089w**
**Chemical Abstracts, vol. 99, no. 25, 19. December 1983, Columbus, Ohio, (US), Kawai Koichi: "Effect of cysteamine on canine splanchnic D cells", p. 154, abstract no. 206966x**

(73) Patentinhaber: **AMMON, Hermann P.T., Im Kleeacker 30, D-7400 Tübingen (DE)**

(72) Erfinder: **AMMON, Hermann P.T., Im Kleeacker 30, D-7400 Tübingen (DE)**

(74) Vertreter: **Kraus, Walter, Dr. et al, Patentanwälte Kraus, Weisert & Partner Thomas-Wimmer-Ring 15, D-8000 München 22 (DE)**

(56) Entgegenhaltungen: (Fortsetzung)
**Chemical Abstracts, vol. 96, no. 7, 15 February 1982, Columbus, Ohio, (US), H.P.T. Ammon et al.: "Potentation of the insulin-releasing capacity of tolbutamide by thiols: studies on the isolated perfused pancreas", p. 45, abstract no. 46096w**
**Unlisted Drugs, vol. 24, no. 5, May 1972, Chatham (N.J.),(US), p. 68:b**

**Die Akte enthält technische Angaben, die nach dem Eingang der Anmeldung eingereicht wurden und die nicht in dieser Patentschrift enthalten sind.**

**EP 0 165 274 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Der Diabetes (Zuckerkrankheit) kann in verschiedenen Formen auftreten. Der sogenannte Jugend-Diabetes (Diabetes I) ist gekennzeichnet durch das Unvermögen der dafür an sich zuständigen Langerhans'schen Inseln in der Bauchspeicheldrüse zu ausreichender Produktion und Abgabe des für die Elimination von Glucose aus dem Blut erforderlichen Insulins. Das Insulin muß zugegeben werden. Solche Patienten sind «insulinpflichtig». Anders verhält es sich bei dem sogenannten Alters-Diabetes (Diabetes II): Er ist gekennzeichnet durch das Unvermögen, das an sich in den Zellen der Langerhans'schen Inseln vorhandene Insulin an das Blut abzugeben. Solche Patienten sind zwar nicht «insulinpflichtig». Ihre Behandlung erfolgt durch Diät und ferner durch Stoffe, die die Insulinsekretion aktivieren. Hierfür bekannte Wirkstoffe sind z.B. die Sulfonylharnstoffe. Sie aktivieren die Insulinsekretion über einen speziellen Mechanismus direkt und/oder zusammen mit Blutzuckeranstieg.

Es ist bekannt, daß zwischen dem Thiolgehalt der Zellen der Langerhans'schen Inseln und der durch Glucose induzierten Insulinsekretion ein Zusammenhang besteht. Dabei ergab sich, daß das in den Langerhans'schen Inseln natürlich enthaltene Tripeptid Glutathion ein Redox-System bildet und daß eine Korrelation besteht zwischen dem Verhältnis von reduziertem Glutathion (GSH) zu oxidiertem Glutathion (GSSG) und der Konzentration an Glucose bei der induzierten Insulinsekretion (Ammon et al, Diabetes 29 (1980), No. 10 S. 830–834). Des weiteren spielt das Redox-Paar NADP/NADPH (Nikotinamid-adenindinucleotidphosphat), das als Wasserstoffüberträger dient, eine Rolle.

Es ist ferner bekannt, daß Agenzien, die NADPH und GSH oxidieren, die durch Glucose induzierte Insulinsekretion inhibieren (Ammon et al., Arch. Pharmakol. 207 (1979), S. 91–96; Ammon et al., Endocrinology, Vol. 112, No. 2 (1983), 720–726).

Andererseits ergab eine Addition von GSH und Cystein in vitro eine Erhöhung der durch Glucose stimulierten Insulinsekretion (Ammon et al, Arch. Pharmakol. 317 (1981), S. 262–267). Weitergehende Untersuchungen haben gezeigt, daß durch die Hinzufügung von GSH und Cystein in vitro jedoch ohne eine für die Stimulanz genügend hohe Konzentration an Glucose keine Insulinsekretion herbeigeführt wurde. Aus all diesen Beobachtungen wurde das nachfolgend stilisiert dargestellte Modell über die Wirkung der erwähnten Redox-Paare postuliert. Es geht davon aus, daß die Permeabilität der β-Zelle einer Langerhans'schen Insel im Sinne verstärkter oder verminderter Insulinsekretion von dem Redoxzustand der Membran-SH-Gruppen abhängig ist.

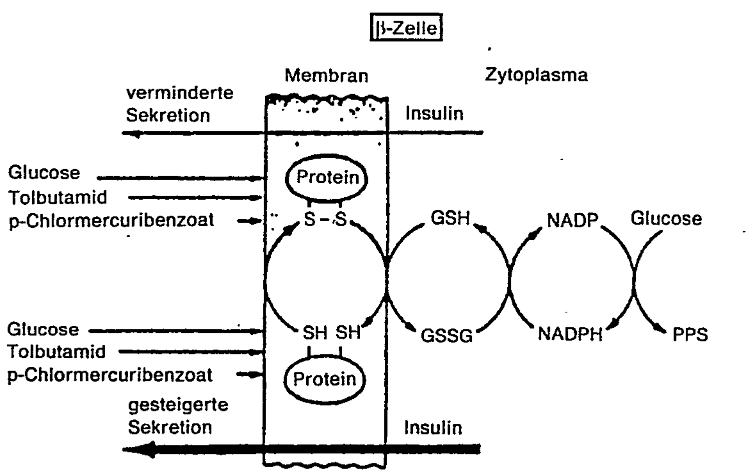


Das Protein der Membrane ermöglicht bei reduziertem Zustand der SH-Gruppen eine gesteigerte Sekretion von Insulin, während im oxidierten Zustand nur eine geringe Sekretion möglich ist. Diese Reduktion der Membran-S-S-Gruppen unter Wasserstoffübertragung kann durch das GSH unter Bildung von oxidiertem GSSG geschehen, das seinerseits wieder durch das Redox-Paar NADP/NADPH durch Wasserstoffübertragung in GSH übergeführt werden kann. Das durch diese Wasserstoffübertragung gebildete NADP kann wiederum durch Wasserstoffübertragung aus dem Glucose – PPS – (Pentosephosphatshunt) Weg in die reduzierte Form NADPH übergeführt werden.

Die Stimulation der gesteigerten Insulinsekretion führt also über folgenden Weg:

Glucose → PPS-Weg → NADPH → GSH → reduzierte Membran-SH-Gruppen. Glucose steigert sowohl den Gehalt der Langerhans'schen Inseln an reduzierten Glutathion (GSH) als auch die Sekretion von Insulin, während die externe Zugabe von Insulin zur Abnahme des intrazellularen SH-Gehaltes und zur Hemmung der Sekretion von Insulin führt. Die externe Zufuhr von GSH potenziert die insulinfreisetzende Wirkung von Glucose, dagegen hatte GSH in Abwesenheit von Glucose keine Wirkung.

Im Jahre 1969 wurde von Takehisa Chiba eine Arbeit veröffentlicht, mit dem Titel: «Effect of sulfur-containing compounds on experimental diabetes. VI. Screening of hypoglycemic action of sulfur

containing compounds» (Chem. Abstracts, Bd. 71, Nr. 23, S. 200, 111 089w).

In dieser Arbeit wurde der Einfluß einer ganzen Reihe chemischer Verbindungen, die über freie Thiolgruppen verfügen, auf den Blutzuckerspiegel von Ratten untersucht, die vorher mit Hilfe einer Behandlung von Alloxan diabetisch (hyperglykämisch) gemacht worden waren. Alloxan zerstört die insulinproduzierenden Zellen; daraufhin kommt es zur Hyperglykämie. Die Stoffe wurden intravenös verabreicht, und zwar in Dosen von 100 mg/kg, in einigen Fällen waren es auch 25 bzw. 50 mg/kg. Unter diesen Substanzen waren von den Cystein-Derivaten: Glutathion, L-Cystein, DL-Homocystein und Penicillamin. Die übrigen Verbindungen enthielten kein Cystein im Molekül. Aus den Versuchsergebnissen geht hervor, daß SH-gruppenhaltige Substanzen bei alloxandiabetischen Ratten den Blutzuckerspiegel in geringem Maße senken (10 bis 20%). Dabei war das Vorhandensein einer freien SH-Gruppe Voraussetzung. Die Wirkung dieser Substanzen auf eine Freisetzung von Insulin wurde nicht untersucht; eine solche Untersuchung dürfte auch nur zu negativen Ergebnissen geführt haben, da bei den Versuchstieren die Insulin enthaltenden Zellen durch Alloxan zerstört werden. Die verwendeten Dosen waren im allgemeinen sehr hoch und zum Teil auch toxisch. Eine Unterscheidung in der Wirkung zwischen Cystein-Derivaten und anderen Thiolen konnte nicht getroffen werden, da im Gegensatz zu den Ergebnissen, die der vorliegenden Anmeldung zugrundeliegen, eine blutzuckersenkende Wirkung auch bei Nichtcysteinen beobachtet wurde. Darüber hinaus ist eine blutzuckersenkende Wirkung von 10 bis 20% bei so hohen Dosen an Thiolen für eine antidiabetische Therapie irrelevant.

H. P. T. Ammon und M. Abdel-Hamid (Chem. Abstracts, Bd. 96, Nr. 7, S. 45, 46 096w) untersuchten, ob der Mechanismus der durch Tolbutamid (Sulfonylharnstoff als orales Antidiabetikum) bzw. Glucose induzierbaren Insulinsekretion etwas mit dem Redoxzustand von Thiolen zu tun hat. Als Methode, um diese Fragestellung zu beantworten, wurde hier die Zugabe von Glutathion und L-Cystein gewählt. Es konnte gezeigt werden, daß durch 1 mM Glutathion die durch Tolbutamid und Glucose induzierte Freisetzung von Insulin weiter gesteigert wurde. Im Falle des Cysteins (5 mM) wurde die durch Tolbutamid induzierte Insulinsekretion zwar weiter gesteigert, jedoch trat bei dieser Dosierung von Cystein sogar eine Hemmung der glucoseinduzierten Insulinsekretion auf. Diese Befunde legten nahe, daß zumindest Glutathion etwas mit dem Sekretionsmechanismus von Tolbutamid und Glucose zu tun hat. Hinderlich war allerdings der Befund, nach dem Cystein die durch Glucose induzierbare Insulinsekretion in diesem System eher hemmte. Eine Gesetzmäßigkeit hinsichtlich «Cystein-Derivaten» konnte daher aus dieser Publikation noch nicht erkannt werden. Cystein scheidet als solches wegen der hohen Dosis (5 mM), aber auch wegen seiner offensichtlich toxischen Wirkung, die durch die hohe Dosierung bedingt war, als Substanz aus, die man zur Steigerung der Insulinsekretion verwenden konnte. Es sollte auch betont werden, daß die Konzentrationen an Glutathion in dieser Arbeit noch um ein 100faches höher waren als diejenigen, die erfindungsgemäß verwendet werden.

In einer Arbeit von Kawai Kolchi «Effect of cysteamine on canine splanchnic D-cells» (Chem. Abstracts, Bd. 99, Nr. 25, S. 154, 206 966x) wird berichtet, daß die intravenöse bzw. orale Zufuhr von Cysteamin (Dosen sind nicht angegeben; Cysteamin dient in der Wissenschaft als Werkzeug, mit dem man selektiv die das Hormon Somatostatin enthaltenden D-Zellen der Langerhans'schen Inseln zerstören kann) bei erwachsenen Hunden den Seruminsulinspiegel und den Glucagonspiegel erhöhte. Wenn die Bauchspeicheldrüsen von mit Cysteamin behandelten Hunden herausgenommen wurden und aus ihnen die sezernierenden Langerhans'schen Inseln isoliert wurden, so zeigten diese eine normale Insulinsekretion. Die Zunahme des Seruminsulins kommt bei hohen Dosen Cysteamin dadurch zustande, daß diese Substanz wegen ihrer Wirkung auf die D-Zellen die physiologische Hemmwirkung von Somatostatin auf die Insulinsekretion aufhebt. Eine solche Wirkung ist vom Cystein und seinen Derivaten nicht bekannt.

In der DE-A-2 835 000 wird die Verwendung von N-Acetyl-L-Cystein bei der Behandlung bakterieller Erkrankungen beschrieben. Die Verwendung von Tabletten mit L-Cysteinmethylester · HCl ist aus Unlisted Drugs, Bd. 24, Nr. 5, S. 68 (b) bekannt.

Der vorliegenden Erfindung liegt die Aufgabe zugrunde, eine neue Verwendung von Cystein-Derivaten oder deren Salzen zur Verfügung zu stellen, wobei die Insulinsekretion in Anwesenheit von Glucose angeregt werden soll. Unter Zugrundelegen des oben angegebenen Wirkungsmechanismus soll die Verwendung solcher Stoffe zur Verfügung gestellt werden, die den Gehalt der β-Zelle an reduzierten Thiolgruppen erhöhen. Sie sind damit in der Lage, die Funktion des natürlicherweise in der β-Zelle der Langerhans'schen Insel vorkommenden reduzierten Glutathions einzunehmen, und zwar dadurch, daß sie den Redoxzustand membranständiger SH-Gruppen der β-Zelle in Richtung auf die reduzierte Seite verschieben.

Gegenstand der Erfindung ist die Verwendung von Cystein-Derivaten oder deren Salzen der Formel

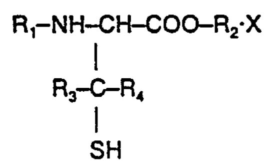

wobei $R_1$ ein Wasserstoffatom oder ein Acetylrest, $R_2$ ein Wasserstoffatom, ein Methyl- oder Ethylrest, $R_3$ ein Wasserstoffatom oder ein Methylrest, $R_4$ ein Wasserstoffatom oder ein Methylrest sowie X eine Protonsäure sind, wobei Cystein ausgenommen ist, zur Herstellung eines Arzneimittels für die Steigerung der durch Glucose induzierten Insulinsekretion der Langerhans'schen Inseln in der Bauchspeicheldrüse.

Überraschenderweise zeigte sich, daß bei der Verwendung dieses Substanzen bei Zugabe in vitro bei Anstieg der Glucosekonzentration über einen gewissen Schwellwert ein Anstieg der Insulinkonzentration erfolgt.

Die erfindungsgemäß verwendeten Verbindungen, insbesondere N-Acetylcystein, zeigen überraschenderweise per se keine hypoglykämische Wirkung, sie zeigen per se auch keine insulinsteigernde Wirkung. Sie wirken auf die Insulinsekretion nur im Zusammenhang mit einer erhöhten Glucosekonzentration. Dies ist ein gravierender Unterschied gegenüber dem oben genannten Stand der Technik, insbesondere gegenüber den Cystein-Derivaten. Die im Handel befindlichen oralen Antidiabetika wirken ihrerseits bereits bei normalem Blutzuckerspiegel hypoglykämisch und setzen Insulin frei und führen dabei oft zu schweren Hypoglykämien als unerwünschte Nebenwirkung. Bei den erfindungsgemäß verwendeten Verbindungen ist dies nicht der Fall.

Als ein Maß für die Wirksamkeit kann der aus der Pharmakologie gebräuchliche Begriff der sogenannten «mittleren effektiven Dosis» $ED_{50}$ in leicht abgewandelter Form benutzt werden. $ED_{50}$ ist diejenige Menge an zugegebenem Thiol, gemessen in millimolarer Konzentration pro Reaktionslösung, die nötig ist, um zu 50% der maximal erreichbaren Zunahme an Insulin zu gelangen. Der $ED_{50}$-Wert ist also ein Maß für die erforderliche Dosis.

Auszugehen ist von einem $ED_{50}$-Wert der reduzierten Form des Glutathions (GSH) von 0,01. Von den erfindungsgemäßen Substanzen zeigen das Cysteamin mit einem $ED_{50}$-Wert von 0,01 und das N-Acetyl-L-Cystein (NAC) mit einem $ED_{50}$-Wert von 0,02 die besten Wirkungen. Daher sind diese Substanzen besonders vorteilhaft. Im einzelnen ergeben sich die $ED_{50}$-Werte für die einzelnen Substanzen aus der folgenden Tabelle:

Tabelle

| Thiol | ~ $ED_{50}$-Wert |
|---|---|
| Glutathion (GSH) | 0,01 |
| Cysteamin $NH_2-CH_2-CH_2-SH$ | 0,01 |
| N-Acetyl-Cystein (NAC)<br>$CH_3-CO-NH-CH-COOH$<br>\|<br>$CH_2$<br>\|<br>SH | 0,02 |
| D-Penicillamin $NH_2-CH-COOH$<br>\|<br>$CH_3-C-CH_3$<br>\|<br>SH | 0,2 |

Tabelle (Fortsetzung)

| Thiol | ~ $ED_{50}$-Wert |
|---|---|
| L-Cystein-methylester · HCL<br>$NH_2-CH-COOCH_3 \cdot HCL$<br>\|<br>$CH_2$<br>\|<br>SH | 0,4 |
| L-Cystein-ethylester · HCL<br>$NH_2-CH-COOC_2H_5 \cdot HCL$<br>\|<br>$CH_2$<br>\|<br>SH | 0,5 |
| N-Acetyl-D-Penicillamin<br>$CH_3-CO-NH-CH-COOH$<br>\|<br>$CH_3-C-CH_3$<br>\|<br>SH | 1,0 |

Bei N-Acetyl-L-Cystein und den anderen Cysteinderivaten handelt es sich um einen grundsätzlich anderen Wirkungsmechanismus als bei den bekannten Sulfonylharnstoffen. Darin liegt zunächst an sich bereits ein Vorteil, da man bestrebt ist, eine bestimmte Krankheit auch durch Pharmaka mit unterschiedlichen Wirkungsmechanismen therapieren zu können. Das ermöglicht es, wenn ein Weg aus anderweitigen Gründen nicht begangen werden kann, auf einen weiteren Weg auszuweichen. Ein weiterer erheblicher Vorteil ist es, daß das N-Acetyl-L-Cystein und andere Cysteinderivate keine Eigenwirkung auf die Insulinsekretion ausüben, sondern nur dann eine Wirkung hervorrufen, wenn die Glucosekonzentration einen bestimmten Schwellwert überschritten hat. Es wirkt also als «Verstärker» bei Auftreten der im Normalfall die Insulinsekretion stimulierenden Bedingungen (Anwesenheit von Glucose). Es ist also nicht zu befürchten, daß eine Glucoseelimination aus dem Blut auch dann durch verstärkte Insulinsekretion herbeigeführt wird, wenn die Glucosekonzentration noch nicht über dem Normalwert liegt. Das bedeutet, daß keine hypoglykämischen Nebenwirkungen zu befürchten sind.

Im übrigen verstärken das N-Acetyl-L-Cystein bzw. Cysteinderivate die Wirkung des bekannten Sulfonylharnstoffes Tolbutamid. Beide Substanzen potenzieren sich gegenseitig. Das N-Acetyl-L-Cystein besteht aus zwei physiologisch leicht metabolisierbaren Grundbausteinen. Es ist also eine Substanz, von der kaum zu befürchten ist, daß sich unerwünschte Nebenwirkungen ergeben.

Die Applikation kann jeweils vor der Nahrungsaufnahme erfolgen; die Wirkung setzt erst mit der Nahrungsaufnahme, d.h. mit Anstieg der Glucosekonzentration im Blut ein und endet auch, wenn die Glucose auf den Normalwert zurückgegangen

ist. Ferner ergibt sich, daß man hinsichtlich der Dosierung mit vernünftigen Mengen auskommt. Die Bereitschaft zur Wirksamkeit bleibt im Körper über einen gewissen Zeitraum unverändert erhalten; innerhalb dieses Zeitraumes, der mindestens 30–60 Minuten beträgt, kann Nahrungsaufnahme erfolgen, in deren Gefolge ein Anstieg der Glucosekonzentration auftritt, der dann durch die verstärkte Insulinsekretion, wie sie durch das N-Acetyl-Cystein angeregt wird, abgebaut wird.

Im Hinblick auf diese besonderen Eigenschaften wurde das N-Acetyl-L-Cystein auch in vivo untersucht. Das rechtfertigt den Schluß, daß bei den anderen Substanzen die «in vitro» gefundenen Eigenschaften ebenfalls «in vivo» anzutreffen sind. Es handelt sich bei dieser Gruppe von Substanzen generell um Stoffe, von denen man weiß, daß sie nicht spezies-spezifisch sind, so daß auf der Grundlage der im folgenden dargestellten Versuche auch die Wirkung beim Menschen anzunehmen ist.

Die Erfindung wird auch nicht dadurch nahegelegt, daß N-Acetyl-L-Cystein für eine andere Indikation bereits bekannt ist. Man verwendet es seither als schleimlösendes Mittel bei der Mucoviscidosis und als Leberschutzfaktor. Aus dieser Indikation läßt sich nicht schließen, daß es auch für den hier in Anspruch genommenen Zweck, nämlich die Stimulation der Insulinsekretion der Langerhans'schen Inseln der Bauchspeicheldrüse, und damit zur Therapie von Diabetes II, wirksam ist.

Die Erfindung wird auch dann verwirklicht, wenn man die SH-Gruppe mit einer intracellular abspaltbaren Schutzgruppe versieht.

Im folgenden sind einige Ausführungsbeispiele der Erfindung näher beschrieben. Es zeigen:

Figur 1–10 den Einfluß von verschiedenen Thiolen auf die durch Glucose induzierte Insulinsekretion Langerhans'scher Inseln in vitro;

Figur 11 den Einfluß des N-Acetyl-L-Cysteins auf die durch Glucose induzierte Insulinsekretion in vivo;

Figur 12 den Einfluß des N-Acetyl-L-Cysteins bei zeitlicher Zugabe von der Glucose;

Figur 13 den zeitlichen Verlauf der Glucosekonzentration, gemessen in mM/l, im Plasma von Ratten, bei Zugabe von N-Acetyl-L-Cystein.

In allen Diagrammen sind die statistischen Mittelwerte der mehrfach durchgeführten (n) Versuche eingetragen. Zur statistischen Wertung wurde der sogenannte «Student's t-Test» benützt. Um vergleichende Betrachtungen der einzelnen Meßergebnisse durchführen zu können, wurde die «mittlere, effektive Dosis» $ED_{50}$ bestimmt, d.h., diejenige Dosis, die 50% des maximalen therapeutischen Effekts hervorruft. Das ist exakt diejenige Menge Thiol, die nötig ist, um 50% der maximalen Insulinsekretion zu erreichen.

Bei den in Figur 1–3 aufgezeigten Ausführungsbeispielen zeigt das N-Acetyl-Cystein (NAC) mit seinem $ED_{50}$-Wert von ungefähr 0,02 gegenüber den Ester-Hydrochloriden (Methylester 0,4, Ethylester 0,5) die beste Wirkung bezüglich der gesteigerten induzierten Insulinsekretion.

Die Tiere beiderlei Geschlechts wurden aus einer lokalen Zucht entnommen. Sie wogen ca. 300 g und wurden mit einer Standard-Diät (Altromin) ernährt. Die Langerhans'schen Inseln wurden nach Lasy und Kostianovsky (Diabetes 16 (1967), S. 35–39) isoliert. Die Insulinbestimmung erfolgte nach Söldner und Slone (Diabetes 14 (1965) S. 771–779). Glucose und Thiol wurden dabei nahezu gleichzeitig verabreicht (Differenz 30 Sek.). Als Ordinate ist der Insulingehalt pro ml Untersuchungslösung pro 5 Langerhans'scher Inseln nach einer Zeit von 10 Minuten aufgetragen. Als Einheit der grafischen Darstellung dienen «Micro Units of Immunoreactive Insulin» (1UIRI). Als Abszisse ist die Zugabe Thiol in mM pro Volumen Untersuchungslösung angegeben.

Die Figuren 1–3 zeigen den Einfluss von N-Acetyl-L-Cystein (NAC), L-Cystein Methylester · HCl, sowie von L-Cystein-Ethylester · HCl auf eine von 11,1 mM glucoseinduzierte Insulinsekretion der Langerhans'schen Inseln von Ratten.

Figur 4 und 5 zeigen die Untersuchungsergebnisse zweier weiterer Cystein-Derivate, nämlich das D-Penicillamin und das N-Acetyl-D-Penicillamin sowie unter Figur 6 das Cysteamin. In diesen Diagrammen zeigt die gestrichelt eingezeichnete Linie die Ergebnisse bei einer Glucosekonzentration von 2,8 mM, die ihrerseits die Sekretion von Insulin nicht stimuliert, und die durchgezogene Linie die Ergebnisse bei einer Glucosekonzentration von 11,1 mM. Die $ED_{50}$-Werte ergaben für das D-Penicillamin 0,2 für das N-Acetyl-D-Penicillamin 1,0 und für das Cysteamin den beachtlichen Wert von 0,01.

Die Interpretation der Untersuchungsergebnisse bei geringen Glucosekonzentrationen (2,8 mM gestrichelte Linie) sind dahingehend zu tätigen, daß unter einer gewissen Schwellkonzentration an Glucose bei Zugabe von Cystein-Derivaten, deren Salze oder Cysteamin keine erhöhte Insulinsekretion erfolgt. Dies bedeutet, besonders in Hinsicht auf eine spätere pharmakologische Anwendung übersetzt, daß erst ein Übersteigen einer gewissen Blutzuckerkonzentration wie sie z.B. nach einer Nahrungsaufnahme erfolgt, die induzierte Insulinsekretion folgt.

Figur 7 bis 10 zeigen zur Abgrenzung die Untersuchungsergebnisse von cystein-ähnlichen Substanzen, die ebenfalls Thiolgruppen enthalten. Das Homocystein (vgl. Figur 7) unterscheidet sich vom Cystein nur durch den Einschub einer $CH_2$-Gruppe; es ist also dem Cystein nahe verwandt, allerdings kein Derivat. Bei ihm ergibt sich keine Einwirkung auf die durch Glucose induzierte Insulinsekretion.

Figur 10 zeigt die Wirkung des 2-Mercaptoäthansulfonatnatriumsalzes (Mésna), das sowohl eine Thiolgruppe als auch eine Sulfongruppe enthält, so daß aus diesem Grund eine insulinsekretionserfördernde Wirkung zu vermuten war. Eine solche wurde jedoch nicht beobachtet.

Figur 11 zeigt die Untersuchungsergebnisse mit N-Acetyl-L-Cystein bei Versuchen «in vivo» an Ratten. Hier ist die Änderung des Plasmainsulingehaltes gemessen in «Micro Units pro ml» (µ U/ml) gegenüber der Zeit aufgetragen. Die gestrichelte Linie zeigt die Änderung des Plasmainsulins von Ratten, denen zum Zeitpunkt t = 0 0,5 g Glucose pro kg Körpergewicht verabreicht wurde, diese gestrichelte Linie ist die Referenzkurve; sie zeigt das «normale» Verhalten von Ratten ohne zusätzliche Verabreichung von Thiolen.

In Figur 11 zeigen die durchgezogenen Linien den Verlauf bei Ratten, denen zum Zeitpunkt t = 0 eine Dosis von 0,05 g Glucose pro kg Körpergewicht (g/kg KG) verabreicht und 0,5 Minuten (t = −0,5) vor diesem Zeitpunkt 0,1 oder 0,05 oder 0,025 mM NAC/kg KG zusätzlich verabreicht wurden. In Zeitabständen von 5 Minuten (t = 5, 10, 15, 20, 25, 30 Min.) wurde an den Tieren das Plasmainsulin bestimmt. Der Kurvenverlauf zeigt eindeutig die durch NAC gesteigerte Insulinsekretion.

In Figur 12 werden nun Untersuchungen gezeigt, bei denen die Reaktionsbedingungen gegenüber den vorher gezeigten derart abgeändert sind, daß das NAC zeitlich vor der Glucose verabreicht wird. Die Glucose wird zum Zeitpunkt t = 0 in der Dosierung 0,1 mM/kg KG gegeben. Das NAC wird jedoch 30 Min. vor der Glucose verabreicht (t = −30 Min.). Es ist eindeutig, daß NAC allein keine stimulierende Wirkung auf die Insulinsekretion hat, sondern daß erst durch die Zugabe von Glucose der induzierte Mechanismus ins Rollen kommt. Aus dieser Kurve ist eine weitere besonders vorteilhafte erfindungsgemäße Auswirkung zu entnehmen: Vergleicht man nämlich den Gehalt an Plasmainsulin bei der Ratte, der N-Acetyl-Cystein gegeben wurde, mit dem der Ratte ohne Zugabe, jeweils zum Zeitpunkt t = 5 mit dem in Figur 11 im selben Zeitpunkt bei Verabreichung von 0,1 mM NAC/kg KG, so stellt man fest, daß sich kein Unterschied ergibt (~ 160 µU). Das bedeutet, daß das NAC, auch noch, nachdem es bereits 30 Minuten im Körper der Ratte war, dieselbe Wirkung bezüglich der positiv gesteigerten Insulinsekretion hat wie das in Figur 11, das die Wirkung bei NAC nahezu gleichzeitiger (Differenz 30 Sek.) Zugabe der Glucose zeigt. Daraus folgt: Das N-Acetyl-L-Cystein wird also in einem Zeitraum von mindestens 60 Minuten nicht in dem Maße eliminiert, daß seine oben beschriebene Wirkung nicht mehr vorhanden ist. Übersetzt in ein mögliches therapeutisches Konzept bedeutet dies, daß die Applikation eines Medikaments zur Bekämpfung des Diabetes II mit N-Acetyl-L-Cystein als Wirkstoff jeweils vor der Nahrungsaufnahme erfolgen kann, und seine pharmakologische Wirksamkeit bis zu dem dadurch bedingten Blutzuckeranstieg voll beibehält.

Figur 13 zeigt den zeitlichen Verlauf der Änderung der Plasmaglucosekonzentration (mM pro Liter). Dabei zeigt die gestrichelte Linie wiederum die Werte der «Referenzratte» (keine Zugabe von N-Acetyl-L-Cystein), der zum Zeitpunkt t = 0 eine Dosis von 0,05 g Glucose/kg KG verabreicht wurde. Die durchgezogene Linie zeigt den Verlauf bei einer «N-Acetyl-L-Cystein-Ratte» (Zugabe von NAC) der zum Zeitpunkt t = −0,5 zusätzlich 0,025 mM NAC/kg KG verabreicht wurde. Bei der «NAC-Ratte» ist durch den erhöhten Insulinausstoß der Plasmaglucosegehalt bereits zum Zeitpunkt t = 5 wesentlich geringer als bei der «Referenzratte». Der Glucosewert sinkt wesentlich schneller auf den «Normalwert». Dieser Wert wird jedoch nicht unterschritten. Dies bedeutet wiederum, wenn man es in ein mögliches therapeutisches Konzept übersetzt, daß der Blutzuckergehalt bei Nahrungsaufnahme nicht zu stark ansteigt und dann schnell auf seinen «Normalwert» abgesenkt wird. Dabei sinkt er nicht unter den «Normalwert» ab (keine «Unterzuckerung»).

Der eingangs postulierte Wirkungsmechanismus, nämlich die direkte Einwirkung von Glucose und Thiol in die Reaktionsfolge, erhält dadurch weitere Unterstützung. Die bisher verwendeten Medikamente bei der Bekämpfung der Diabetes II zeigen im Gegensatz dazu hypoglykämische Wirkungen. So konnten besonders durch altersbedingte Unaufmerksamkeiten bei nicht vorschriftsmäßiger Dosierung, insbesondere bei Überdosis, oft lebensbedrohende Zustände der Patienten eintreten. Nach der Erfindung steht jetzt ein Medikament ohne diese Gefahren für breite Anwendungsgebiete zur Verfügung.

**Patentansprüche**

1. Verwendung von Cystein-Derivaten oder deren Salzen der Formel

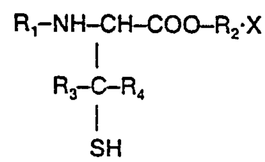

wobei $R_1$ ein Wasserstoffatom oder ein Acetylrest, $R_2$ ein Wasserstoffatom, ein Methyl- oder Ethylrest, $R_3$ ein Wasserstoffatom oder ein Methylrest, $R_4$ ein Wasserstoffatom oder ein Methylrest sowie X eine Protonsäure sind, wobei Cystein ausgenommen ist, zur Herstellung eines Arzneimittels für die Steigerung der durch Glucose induzierten Insulinsekretion der Langerhans'schen Inseln in der Bauchspeicheldrüse.

2. Verwendung von Cysteamin der Formel $NH_2-CH_2-CH_2-SH$ zur Herstellung eines Arzneimittels für die Steigerung der durch Glucose induzierten Insulinsekretion der Langerhans'schen Inseln in der Bauchspeicheldrüse.

3. Verwendung von Cystein-Derivaten oder deren Salzen nach Anspruch 1, dadurch gekennzeichnet, daß das verwendete Cystein-Derivat N-Acetyl-L-Cystein, L-Cystein-Methylester-HCl, L-Cystein-Ethylester-HCl, D-Penicillamin und/oder N-Acetyl-D-Penicillamin ist.

4. Verwendung von Cystein-Derivaten oder deren Salzen nach Anspruch 1 oder 3 oder von Cysteamin nach Anspruch 2, dadurch gekennzeichnet, daß die SH-Gruppe mit einem intracellular abspaltbaren Schutzrest versehen ist, nach dessen Abspaltung die freie SH-Gruppe vorliegt.

## Claims

1. The use of cysteine derivatives or salts thereof corresponding to the following formula

$$\begin{array}{l} R_1\text{–NH–CH–COO–}R_2\cdot X \\ \qquad\quad | \\ \quad\;\; R_3\text{–C–}R_4 \\ \qquad\quad | \\ \qquad\;\; SH \end{array}$$

wherein $R_1$ denotes a hydrogen atom or an acetyl group, $R_2$ denotes a hydrogen atom or a methyl or ethyl group, $R_3$ denotes a hydrogen atom or a methyl group, $R_4$ denotes a hydrogen atom or a methyl group and X stands for a protonic acid, with the exclusion of cysteine, for the preparation of a medicament for increasing the glucose induced insulin secrection of the islets of Langerhans in the pancreas.

2. The use of cysteamine corresponding to the formula $NH_2–CH_2–CH_2–SH$ for the preparation of a medicament for increasing the glucose induced insulin secretion of the islets of Langerhans in the pancreas.

3. The use of cysteine derivatives or salts thereof according to claim 1, characterised in that the cysteine derivative used is N-acetyl-L-cysteine, L-cysteine-methyl ester-HCl, L-cysteine-ethyl ester HCl, D-penicillamine and/or N-acetyl-D-penicillamine.

4. The use of cysteine derivatives or salts thereof according to claim 1 or 3 of of cysteamine according to claim 2, characterised in that the SH group is provided with a protective group which can be split off intracellularly, leaving the free SH group in position after removal of said protective group.

## Revendications

1. Utilisation de dérivés de la cystéine ou de leurs sels répondant à la formule

$$\begin{array}{l} R_1\text{–NH–CH–COO–}R_2\cdot X \\ \qquad\quad | \\ \quad\;\; R_3\text{–C–}R_4 \\ \qquad\quad | \\ \qquad\;\; SH \end{array}$$

$R_1$ étant un atome d'hydrogène ou un reste acétyle, $R_2$ un atome d'hydrogène, un reste méthyle ou éthyle, $R_3$ un atome d'hydrogène ou un reste méthyle, $R_4$ un atome d'hydrogène ou un reste méthyle, et X un acide protonique, à l'exclusion de la cystéine, pour préparer un médicament destiné à accroître la sécrétion d'insuline des îlots de Langerhans du pancréas induite par le glucose.

2. Utilisation d'amine cystéique répondant à la formule $NH_2–CH_2–CH_2–SH$ pour préparer un médicament destiné à accroître la sécrétion d'insuline des îlots de Langerhans du pancréas induite par le glucose.

3. Utilisation de dérivés de la cystéine ou de ses sels selon la revendication 1, caractérisée en ce que le dérivé de la cystéine utilisé est la N-acétyl-L-cystéine, le L-cystéate chlorhydrique de méthyle, le L-cystéate chlorhydrique d'éthyle, la D-pénicillamine et/ou la N-acétyl-D-pénicillamine.

4. Utilisation de dérivés de la cystéine ou de leurs sels selon la revendication 1 ou 3 ou d'amine cystéique selon la revendication 2, caractérisée en ce que le groupe SH comporte un reste protecteur pouvant subir une séparation intracellulaire, après la séparation duquel le groupe SH libre est présent.

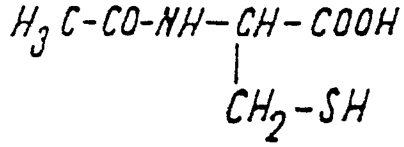

N-Acetyl-L-cystein

$ED_{50} \sim 0{,}02$

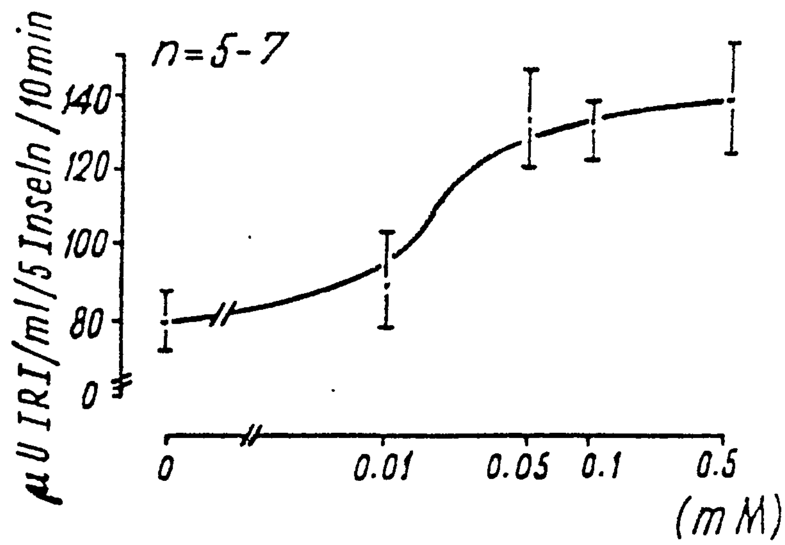


Fig.1

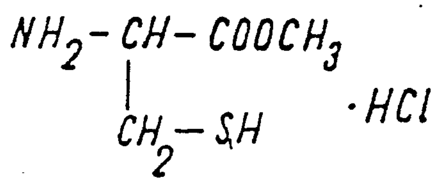

L-Cystein-methylester-HCl

$ED_{50} \sim 0{,}4$

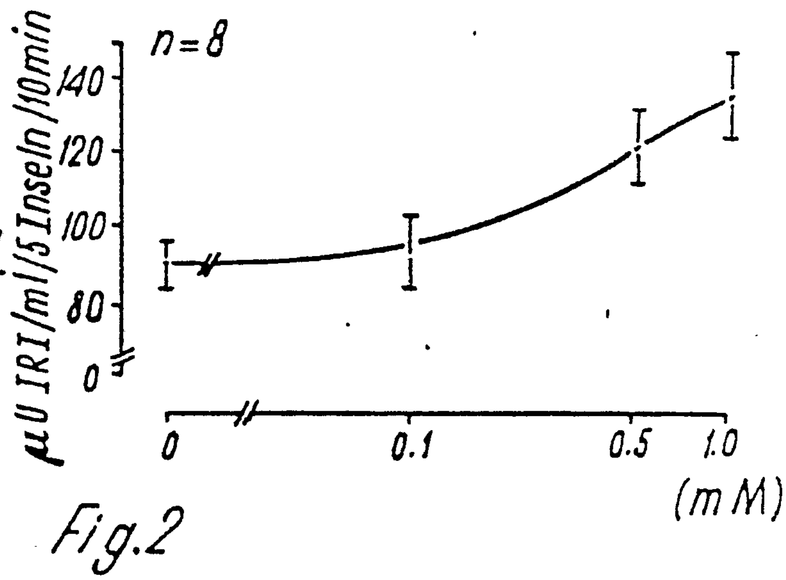


Fig.2

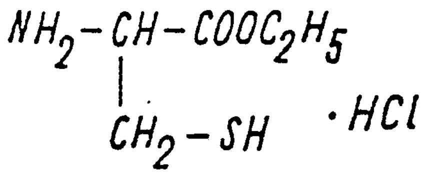

L-Cystein-aethylester-HCl

$ED_{50} \sim 0{,}5$

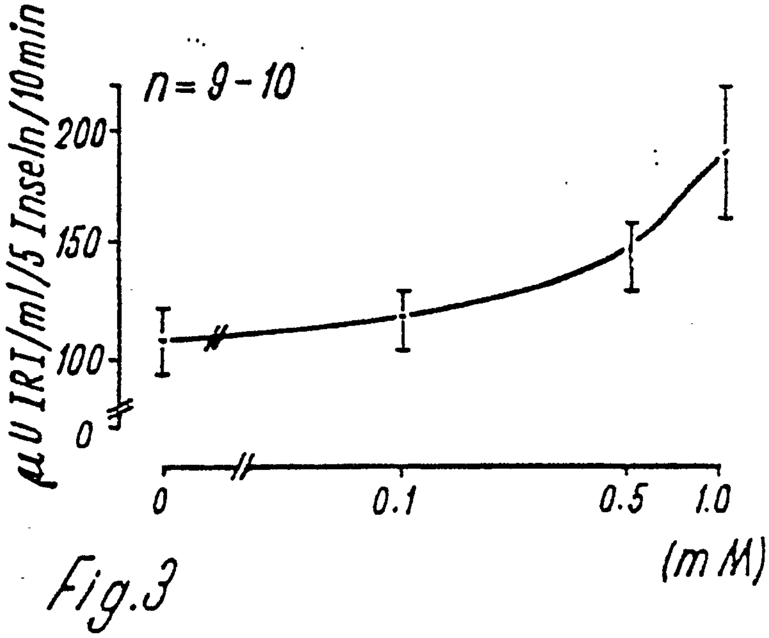


Fig.3

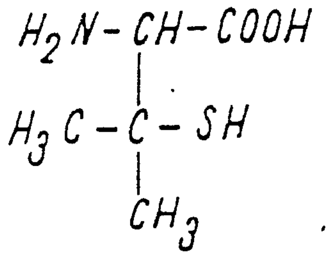

D-Penicillamin

$ED_{50} \sim 0,2$

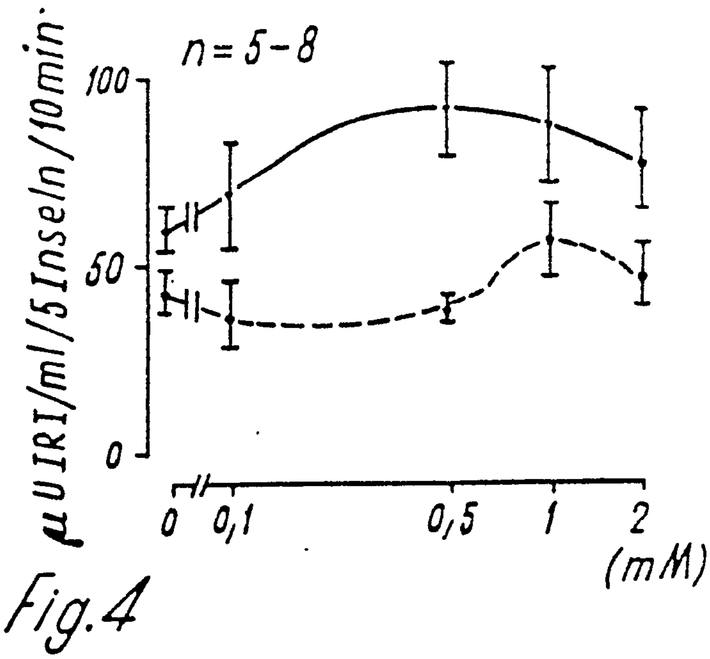


Fig.4

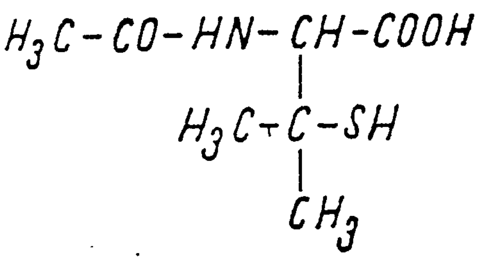

N-Acetyl-D-Penicillamin

$ED_{50} \sim 1,0$

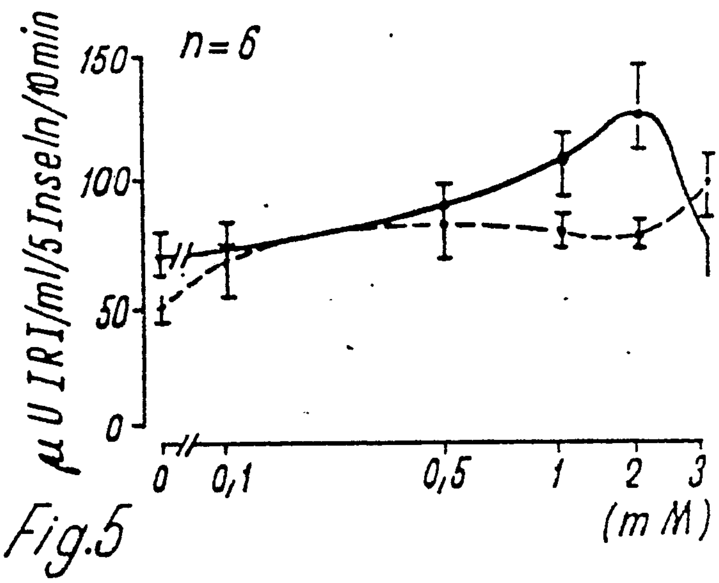


Fig.5

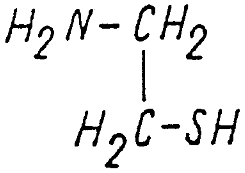

Cysteamin

$ED_{50} \sim 0,01$

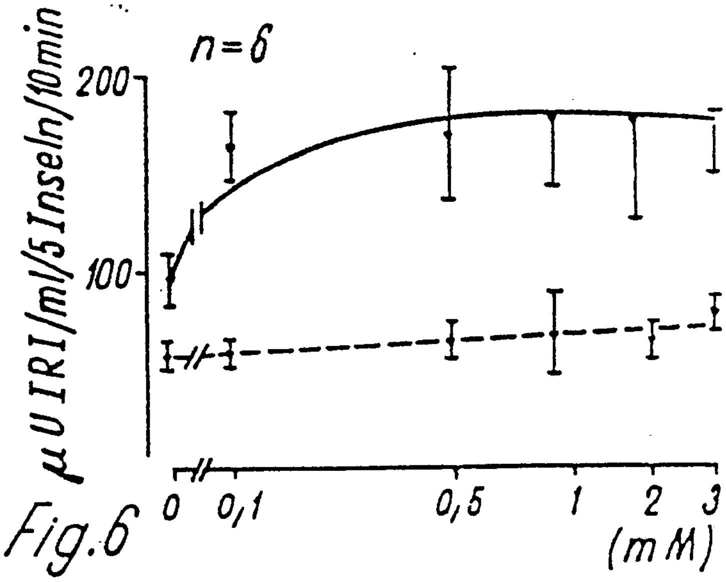


Fig.6

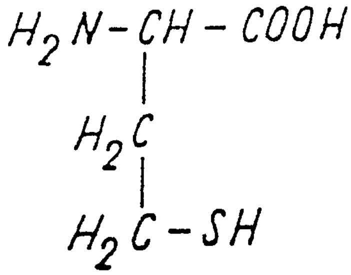

Homocystein

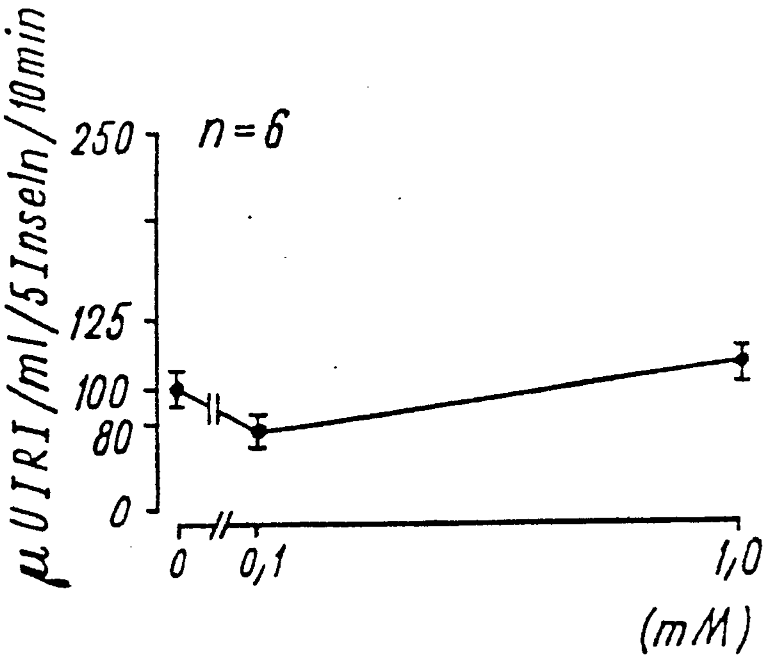


Fig.7

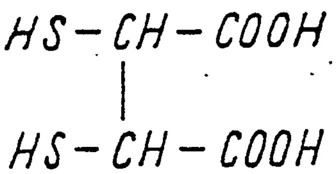

Dimercaptobernsteinsäure

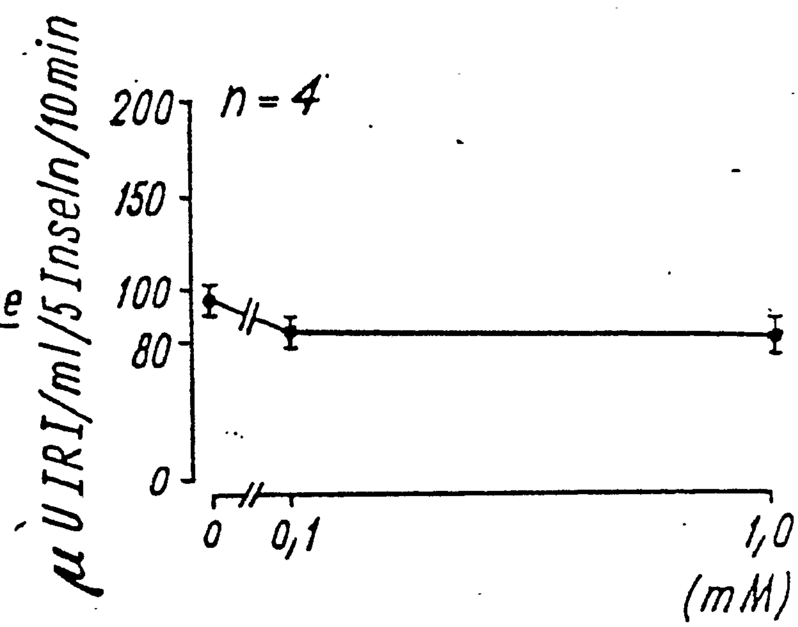


Fig.8

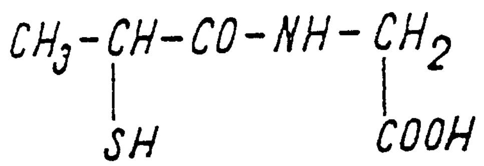

2-Mercaptopropionylglycin
(Thiola)

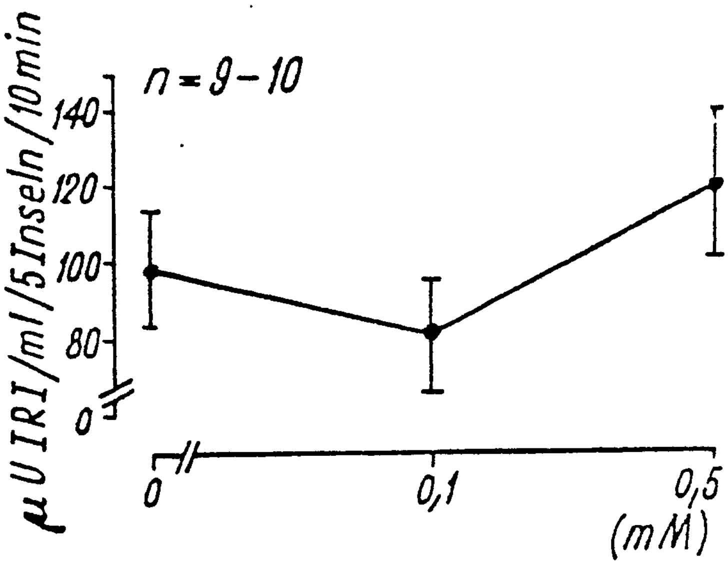


Fig.9

$HS-CH_2-CH_2-SO_3Na$

2-Mercaptoethansulfonat-Na
(Mesna)

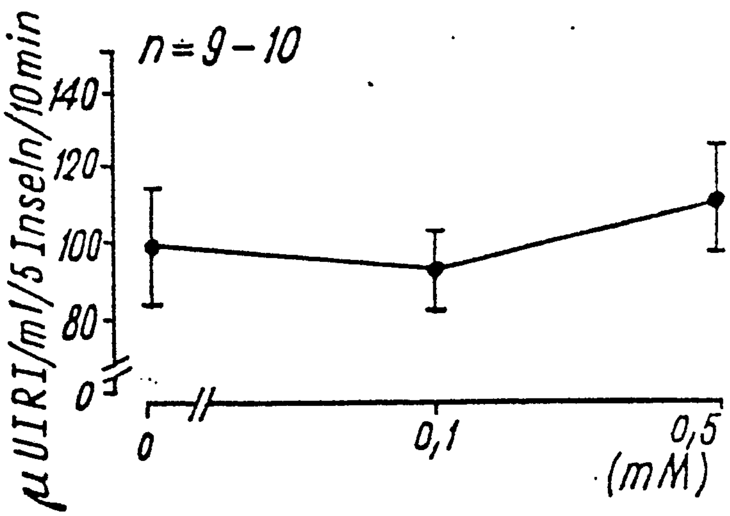


Fig.10

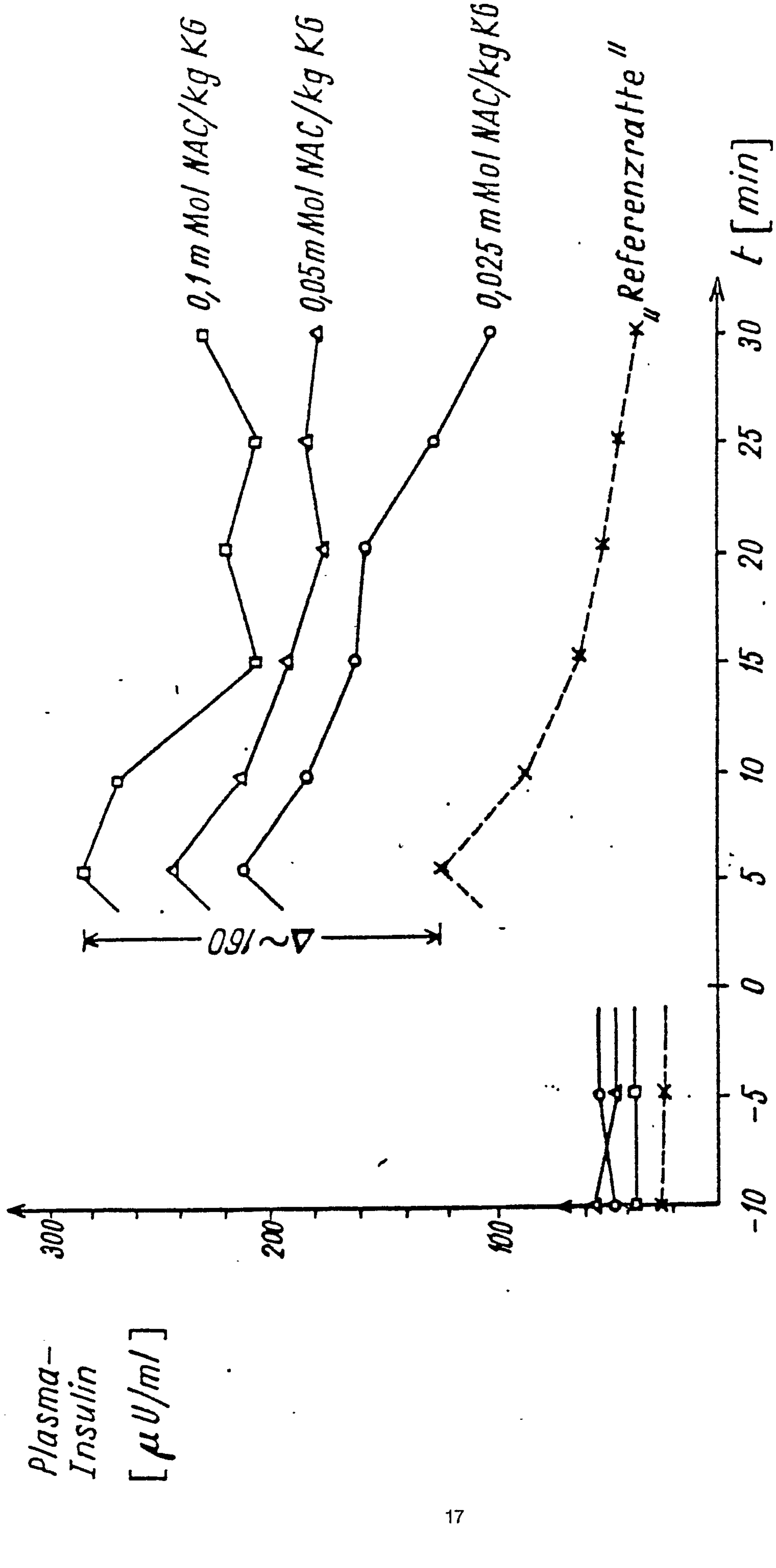
0,1 m Mol NAC/kg KG
0,05 m Mol NAC/kg KG
0,025 m Mol NAC/kg KG
"Referenzratte"
t [min]
Δ~160
Plasma-
Insulin
[µU/ml]
300
200
100
-10
-5
0
5
10
15
20
25
30

Fig.11

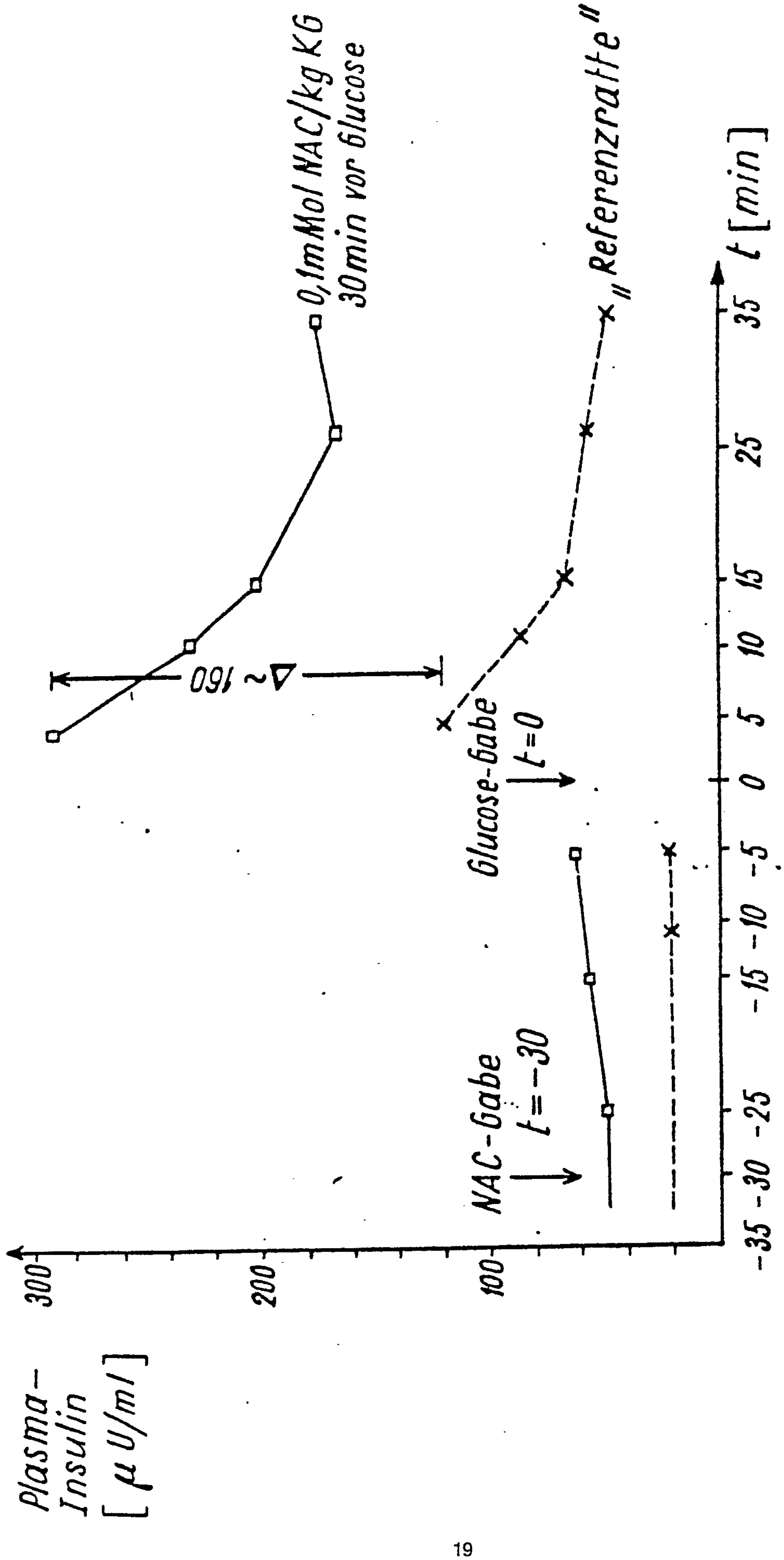

Plasma-Insulin [μU/ml]
300
200
100
-35 -30 -25 -15 -10 -5 0 5 10 15 25 35
t [min]
0,1mMol NAC/kg KG 30min vor Glucose
„Referenzratte"
Δ ~ 160
NAC-Gabe t=-30
Glucose-Gabe t=0


Fig. 12

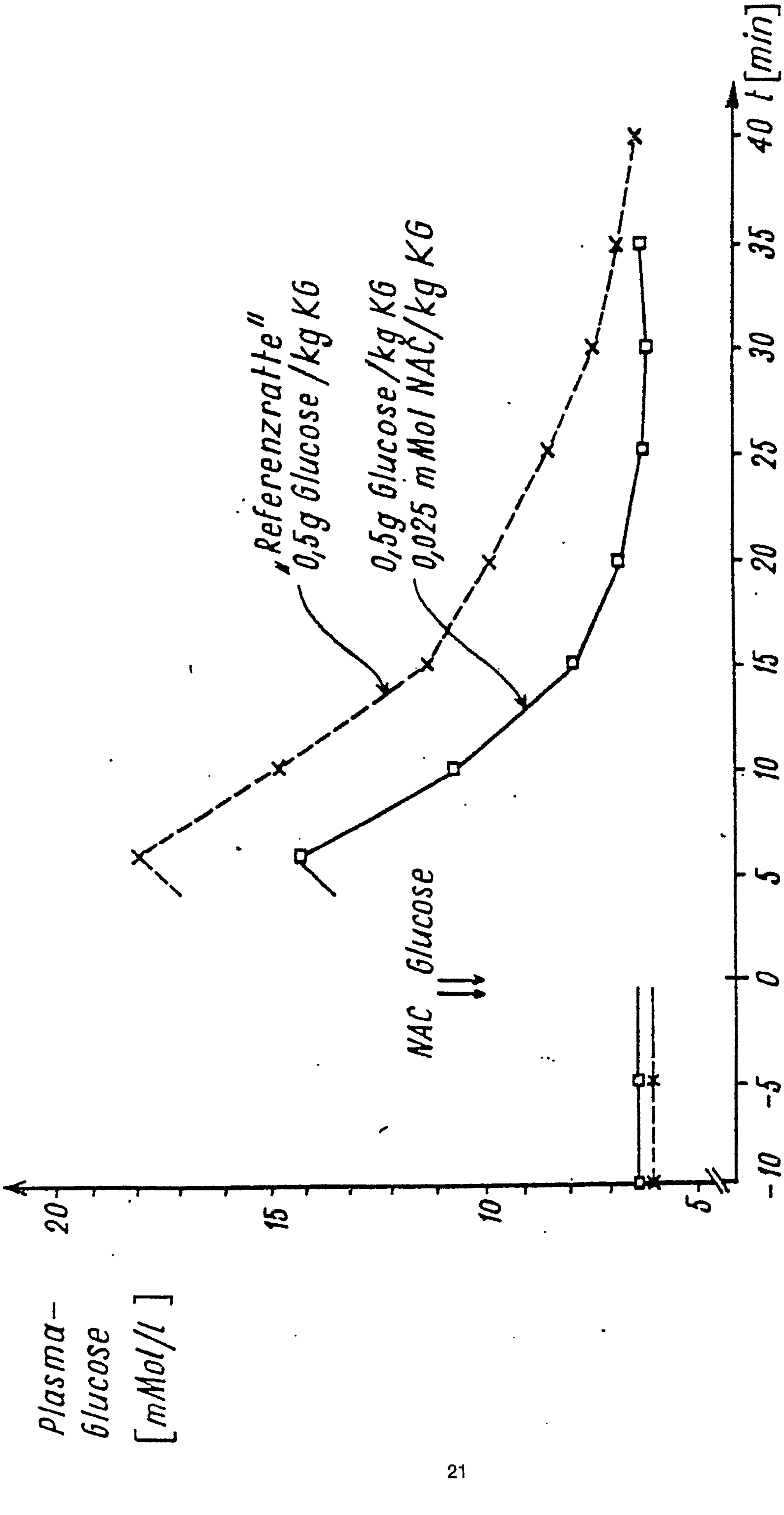

Plasma-
Glucose
[mMol/l]
20
15
10
5
„Referenzratte"
0,5g Glucose/kg KG
0,5g Glucose/kg KG
0,025 mMol NAC/kg KG
NAC
Glucose
-10
-5
0
5
10
15
20
25
30
35
40
t [min]


Fig.13